# EUROPÄISCHE PATENTANMELDUNG

(11) **EP 1 271 344 A1**
(43) Veröffentlichungstag der Anmeldung: **02.01.2003**
(21) Anmeldenummer: 01115234.5
(22) Anmeldetag: 22.06.2001
(51) Int. Cl.: G06F 17/30

(54) **Verfahren und Vorrichtung zum automatischen Verarbeiten und Ausgeben von elektronischen Daten**

(71) Anmelder: Kelman Gesellschaft für Geninformation mbH, 12555 Berlin (DE)
(72) Erfinder: Merkel, Dieter, Dr., 12623 Berlin (DE); Heymann, Stephan, Dr., 10435 Berlin (DE); Rieger, Peter, 10827 Berlin (DE); Wegner, Gunnar, 13156 Berlin (DE)
(74) Vertreter: Liesegang, Eva

(57) **Zusammenfassung**

Die Erfindung betrifft ein Verfahren und eine Vorrichtung zum automatischen Verarbeiten und Ausgeben von elektronischen Daten aus einer Datenspeichereinrichtung. Das Verfahren weist die folgenden Schritte auf: Abrufen einer Folge elektronischer Abfragedaten und der mehreren Folgen elektronischer Testdaten von der Datenspeichereinrichtung zu der elektronischen Verarbeitungseinrichtung; automatisches Verarbeiten der Folge eletronischer Abfragedaten und der mehreren Folgen elektronischer Testdaten in der elektronischen Verarbeitungseinrichtung, um automatisch zu erfassen, welcher Teilfolge der unterteilbaren Folge elektronischer Abfragedaten die mehreren Folgen elektronischer Testdaten jeweils zugeordnet ist; automatisches Erzeugen elektronischer Signale in Abhängigkeit von dem Ergebnis des vorhergehenden Verfahrensschritts, um in einer Ausgabeeinrichtung eine grafische Ausgabe automatisch zu erzeugen, bei der mehreren Folgen elektronischer Testdaten jeweils in einem grafischen Ausgabeteilbereich dargestellt werden, welcher der jeweils zugeordneten Teilfolge der unterteilbaren Folge elektronischer Abfragedaten automatisch zugeteilt ist; Übermitteln der elektronischen Signale an die Ausgabeeinrichtung; und Erzeugen der grafischen Ausgabe auf der Basis der elektronischen Signale in der Ausgabeeinrichtung.

## Beschreibung

Die Erfindung liegt auf dem Gebiet des automatischen Verarbeitens und Ausgebens umfangreicher Mengen elektronischer Daten, die in einer Datenspeichereinrichtung, insbesondere einer Datenbank, abgelegt sind.

Mit der zunehmenden Leistungsfähigkeit elektronischer Computer- bzw. Rechneranlagen werden auf verschiedenen naturwissenschaftlichen/technischen Gebieten umfangreiche Sammlungen elektronischer Daten geschaffen. Die Ansammlung elektronischer Daten umfaßt Informationen, die es dem Benutzer ermöglichen sollen, Problemstellungen auf verschiedenen naturwissenschaftlichen/technischen Gebieten zu lösen. Hierbei besteht in der Regel das Problem, die gesammelten elektronischen Daten mit Hilfe einer elektronischen Verarbeitungseinrichtung, insbesondere einem Computer, so zu verarbeiten und auf der Basis der Verarbeitung elektrischer Ausgabesignale so zu erzeugen, daß es dem Benutzer ermöglicht ist, die ausgegebenen Signale möglichst schnell und effizient für die Problemlösung zu nutzen.

Das beschriebene Problem besteht beispielsweise auch im Zusammenhang mit gespeicherten, elektronischen Daten, die Informationen über mögliche Bindungspartnerschaften zwischen Proteinen umfassen. Hierbei werden sowohl experimentell als auch mit Hilfe computergestützter Rechnungen gewonnene Aussagen über potentielle Protein-Protein-Wechselwirkungen zur Ausbildung einer oder mehrerer Bindungen zwischen einem Test- bzw. Abfrageprotein und möglichen Wechselwirkungsproteinen getroffen. Die Vorhersageergebnisse umfassen hierbei insbesondere eine Aussage über Abschnitte der Aminosäurenfolge des Abfrageproteins, die mit potentiellen Wechselwirkungsproteinen wechselwirken, und Abschnitte der jeweiligen Aminosäurenfolge der Wechselwirkungsproteine, die bei einer möglichen Wechselwirkung mit den Abschnitten der Aminosäurenfolge des Abfrageproteins in Wechselwirkung treten. Dieses bedeutet, daß für die Abschnitte der Aminosäurenfolge des Abfrageproteins und die Abschnitte der jeweiligen Aminosäurenfolge der Wechselwirkungsproteine eine Vorhersage getroffen wird. Die große Anzahl von Proteinen, die bei einer Abfrage nach potentiellen Wechselwirkungspartner für das Abfrageprotein in der Regel gefunden wird, führt dazu, daß die Auswertung der hierbei erzeugten elektronischen Daten für den Benutzer sehr zeitaufwendig ist.

Aufgabe der vorliegenden Erfindung ist es deshalb, ein verbessertes Verfahren und eine verbesserte Vorrichtung zum automatischen Verarbeiten und Ausgeben von elektronischen Daten zu schaffen, die eine anwendungsorientierte Erzeugung und Ausgabe elektronischer Signale auf der Basis der elektronischen Daten ermöglicht.

Nach einem Aspekt der Erfindung ist ein Verfahren zum automatischen Verarbeiten und Ausgeben von elektronischen Daten aus einer Datenspeichereinrichtung mit Hilfe einer elektronischen Verarbeitungseinrichtung geschaffen, wobei die elektronischen Daten eine in Teilfolgen unterteilbare Folge elektronischer Abfragedaten mit einer vorgegebenen Anordnung elektronischer Abfragedatenelemente und mehrere Folgen elektronischer Testdatenelemente mit einer jeweils vorgegebenen Anordnung elektronischer Testdaten umfassen. Das Verfahren weist die folgenden Schritte auf: Abrufen der Folge elektronischer Abfragedaten und der mehreren Folgen elektronischer Testdaten von der Datenspeichereinrichtung zu der elektronischen Verarbeitungseinrichtung; automatisches Verarbeiten der Folge elektronischer Abfragedaten und der mehreren Folgen elektronischer Testdaten in der elektronischen Verarbeitungseinrichtung, um automatisch zu erfassen, welcher Teilfolge der unterteilbaren Folge elektronischer Abfragedaten jede der mehreren Folgen elektronischer Testdaten jeweils zugeordnet ist; automatisches Erzeugen elektronischer Signale in Abhängigkeit von dem Ergebnis des Verfahrensschritts zum Feststellen, um in einer Ausgabeeinrichtung eine grafische Ausgabe automatisch zu erzeugen, bei der die mehreren Folgen elektronischer Testdaten jeweils in einem grafischen Ausgabeteilbereich dargestellt werden, welcher der jeweils zugeordneten Teilfolge der unterteilbaren Folge elektronischer Abfragedaten automatisch zugeteilt ist; Übermitteln der elektronischen Signale an die Ausgabeeinrichtung; und Erzeugen der grafischen Ausgabe auf der Basis der elektronischen Signale in der Ausgabeeinrichtung.

Ein Vorteil, welcher mit der Erfindung gegenüber dem Stand der Technik erreicht ist, besteht darin, daß es für den Benutzer leichter möglich ist, aus einer Vielzahl gespeicherter elektronischer Daten, eine übersichtlich gestaltete Ausgabe auf der Basis automatisch erzeugter, elektronischer Signale zu erhalten, die dem Benutzer das Herausfiltern relevanter Informationen für den Anwendungsfall erleichtern. Dieser Vorteil ergibt sich insbesondere in Verbindung mit der Visualisierung und Auswertung der Vorhersagen von Wechselwirkungen zwischen verschiedenen Proteinen.

Um die automatische Einbeziehung ergänzender, elektronisch gespeicherter Informationen zu ermöglichen, sieht ein zweckmäßige Ausgestaltung der Erfindung vor, daß vor dem Verfahrensschritt zum automatischen Erzeugen der elektronischen Signale unter automatischer Berücksichtigung weiterer elektronischer Informationen eine wenigstens teilweise Überprüfung des Ergebnisses aus dem Verfahrensschritt zum Feststellen in der elektronischen Verarbeitungseinrichtung automatisch ausgeführt wird.

Die Berücksichtigung umfangreicher elektronisch gespeicherter Informationen ist bei einer bevorzugten Ausführungsform der Erfindung dadurch gewährleistet, daß die weiteren elektronischen Informationen zumindest teilweise von externen Datenbanken abgerufen werden.

Um das beschriebene Verfahren in Verbindung mit dem Aufsuchen und Testen der Wechselwirkung zwischen Genabschnitten von Proteinen verwenden zu können, sieht eine vorteilhafte Ausgestaltung der Erfindung vor, daß die Folge elektronischer Abfragedaten eine elektronische Repräsentation einer Abfragegensequenz, die Teilfolgen elektronischer Repräsentationen von Teilgensequenzen der Abfragegensequenz und die mehreren Folgen elektronischer Testdaten elektronische Repräsentationen von Abschnitten von Gensequenzen potentieller Wechselwirkungspartner der Abfragegensequenz sind.

Eine für den Benutzer besonders anschauliche Visualisierung wird bei einer zweckmäßigen Fortbildung der Erfindung dadurch erreicht, daß mit Hilfe der elektronischen Signale grafische Balkenelemte in dem grafischen Ausgabebereich erzeugt werden, die in mehreren Zeilen übereinander ausgegeben werden.

Die Erfindung wird anhand von Ausführungsbeispielen unter Bezugnahme auf eine Zeichnung näher erläutert. Hierbei zeigen:
- Figur 1: ein schematisches Blockschaltbild einer Anordnung zum Ausführen eines Verfahrens zum automatischen Verarbeiten und Ausgeben elektronischer Daten; und
- Figur 2: ein Ausgabebeispiel zur Darstellung potentieller Wechselwirkungsabschnitte zwischen einer Abfragegensequenz und möglichen Protein-Wechselwirkungspartnern;
- Figur 3: einen Teilbereich des Ausgabebeispiels nach Figur 2 mit einem größeren Maßstab.

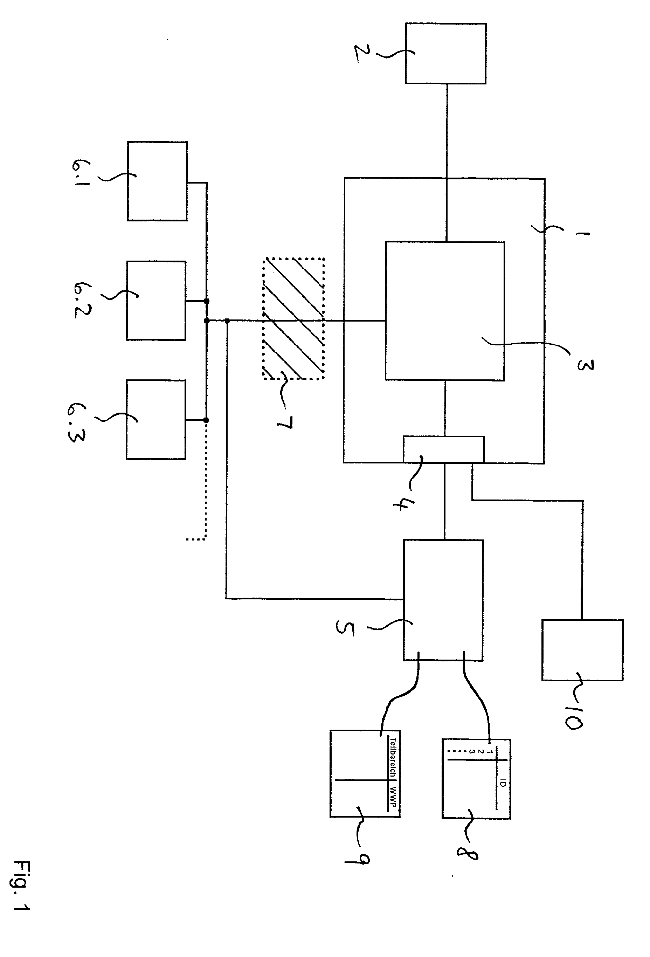

Gemäß Figur 1 ist eine elektronische Verarbeitungseinrichtung 1 mit einer Eingabeinrichtung 2 verbunden. Die elektronische Verarbeitungseinrichtung 1 kann insbesondere ein Computer bzw. eine Rechenanlage sein. Mit Hilfe der Eingabeeinrichtung 2 kann ein Benutzer Eingabebefehle eingeben. Die mit Hilfe der Eingabebefehle erzeugten elektronischen Signale dienen der Steuerung der elektronischen Verarbeitungseinrichtung 1, wobei vorzugsweise eine interaktive Steuerung ausgebildet ist.

Zum Verarbeiten elektronischer Daten und elektronischer Signale weist die elektronische Verarbeitungseinrichtung 1 eine Prozessoreinheit 3 auf. Mit Hilfe der Prozessoreinheit 3 können über eine Schnittstellen- bzw. Abrufeinrichtung 4 elektronische Daten aus einer Speichereinrichtung 5 abgerufen bzw. an die Speichereinrichtung 5 übertragen werden. Die Speichereinrichtung 5 ist in Figur 1 nicht von der elektronischen Verarbeitungseinrichtung 1 umfaßt, kann jedoch als Teil der elektronischen Verarbeitungseinrichtung 1 ausgebildet sein (nicht dargestellt). Bei der Speichereinrichtung 5 kann es sich um eine Festplattenspeicher oder einen anderen geeigneten Speicher zum abrufbaren Speichern elektronischer Daten handeln. Die Speichereinrichtung 5 kann von einer Datenbankeinrichtung umfaßt sein.

Elektronische Signale, die mit Hilfe von Daten erzeugt werden, die über die Schnittstelleneinrichtung 4 von der Speichereinrichtung 5 abgerufen und mit Hilfe der Prozessoreinheit 3 verarbeitet werden, können über eine oder mehrere Ausgabeeinrichtungen 6.1, 6.2, ... ausgegeben werden. Bei den Ausgabeeinrichtungen 6.1, 6.2, ... kann es sich beispielsweise um eine Bildschirmeinrichtung, einen Drucker, einen Plotter oder dergleichen handeln. Es kann auch vorgesehen sein, daß die mehreren Ausgabeeinrichtungen 6.1, 6.2, ... eine weitere Speichereinrichtung umfassen, in welcher die mit Hilfe der Prozessoreinheit 3 erzeugten elektronischen Signale und zugehörige Informationen zur Unterscheidung der Signale als elektronische Daten gespeichert werden.

Gemäß Figur 1 kann auch eine Ausführungsform vorgesehen sein, bei der die mit Hilfe der Prozessoreinheit 3 erzeugten elektronischen Signal, einschließlich zugehöriger Informationen zur Charakterisierung der elektronischen Signale, in der Speichereinrichtung 5 abgelegt werden.

Eine zweckmäßige Ausgestaltung sieht vor, daß die mittels der Prozessoreinheit 3 erzeugten elektronischen Signale von den mehreren Ausgabeeinrichtungen 6.1, 6.2, ... über einen Internetabruf heruntergeladen und anschließend ausgegeben werden. In Figur 1 ist dieses schematisch mit Hilfe eines Blocks 7 dargestellt.

Zur Erläuterung des Zusammenwirkens der Komponenten, die in Figur 1 schematisch als Blockelemente dargestellt sind, wird im Folgenden angenommen, daß in der Speichereinrichtung 5 elektronische Daten gespeichert sind, die elektronische Signale und Informationen betreffend eine Liste 8 von möglichen Wechselwirkungspartnern mit einem Test- bzw. Abfragesequenz umfassen. Bei der Abfragesequenz handelt es sich im vorliegenden Beispiel um eine Folge von Aminosäuren, wobei die Folge von Aminosäuren die Geninformation eines Test- bzw. Abfrageproteins beschreibt.

Mit Hilfe eines experimentellen und/oder eines computer-gestützten Verfahrens wurden Proteine aufgefunden, die potentielle Wechselwirkungspartner für das Abfrageprotein darstellen. Die potentiellen Wechselwirkungspartner sind in der Liste 8 elektronisch gespeichert. Auch die potentiellen Wechselwirkungspartner werden mit Hilfe einer Aminosäurensequenz repräsentiert. Die Wechselwirkung zwischen dem Abfrageprotein und den potentiellen Wechselwirkungspartnern betrifft stets nur Teilbereiche der potentiellen Wechselwirkungspartner und der Abfragesequenz. Dieses bedeutet, daß ein oder mehrere Teilabschnitte der Abfragesequenz, die jeweils eine Anzahl von Aminosäuren umfassen, mit einem jeweiligen Abschnitt eines oder mehrerer der potentiellen Wechselwirkungspartner wechselwirken. Auch der Abschnitt des potentiellen Wechselwirkungspartners wird jeweils von mehreren Aminosäuren gebildet. In der Speichereinrichtung 5 sind deshalb elektronische Daten 9 gespeichert, die Informationen über die potentiellen Wechselwirkungsbereiche umfassen.

Über die Schnittstelleneinrichtung 4 werden die elektronischen Daten 8, 9 aus der Speichereinrichtung 5 durch die Prozessoreinheit 3 abgerufen. Hierbei handelt es sich in der Regel um eine große Anzahl (> 1.000 ?) von potentiellen Wechselwirkungspartner und Wechselwirkungsbereichen. Um die Anzahl der Wechselwirkungspartner bzw. der Wechselwirkungsbereiche einzugrenzen, werden von der Prozessoreinheit 3 über die Schnittstelleneinrichtung 4 weitere elektronisch gespeicherte Informationen, insbesondere aus externen Datenbanken 10 automatische einbezogen. Nach der Verarbeitung der aus der Speichereinrichtung 5 und den externen Datenbanken 10 abgerufenen elektronischen Daten werden mit Hilfe der Prozessoreinheit 3 elektronische Signale automatisch erzeugt, die dann an die Ausgabeeinrichtungen 6.1, 6.2, ... übergeben werden, um in den mehreren Ausgabeeinrichtungen 6.1,.6.2, ... eine Ausgabe automatisch zu erzeugen. Hierbei kann es sich beispielsweise um eine Bildschirmausgabe oder einen Ausdruck mit Hilfe eines Druckers oder eines Plotters handeln.

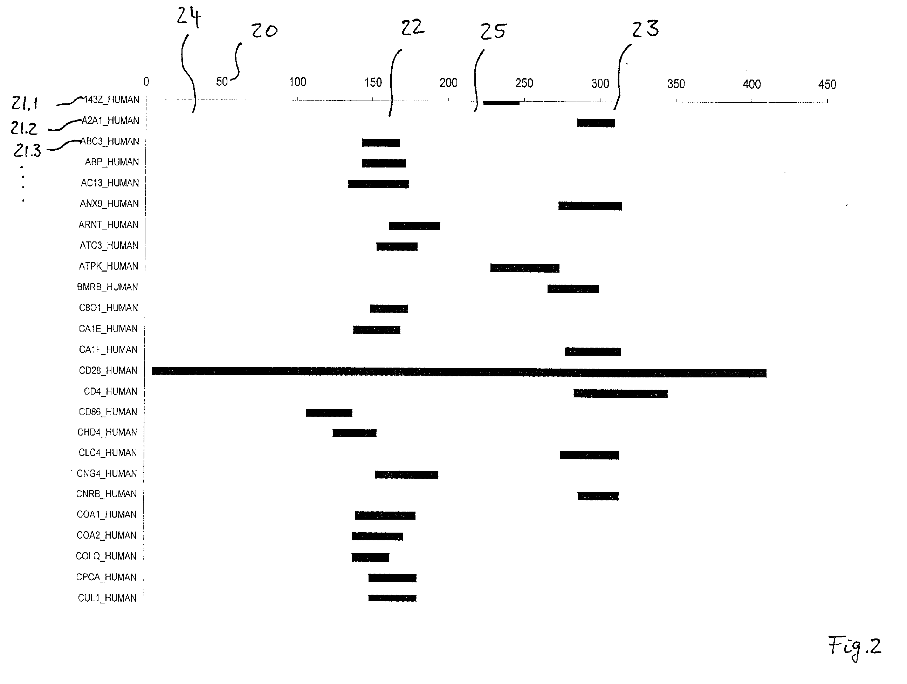

In Figur 2 ist eine auf der Basis der mit Hilfe der Prozessoreinheit 3 erzeugten elektronischen Signale erzeugte Ausgabe schematisch dargestellt. Im unteren Bereich von Figur 2 ist die Folge von Aminosäuren 20 der Abfragesequenz dargestellt, wobei die dargestellten Ziffern die Stellung der jeweiligen Aminosäure in der Gensequenz des Abfrageproteins bezeichnen. Unterhalb der Folge der Aminosäuren der Abfragesequenz 20 sind zeilenweise die potentiellen Wechselwirkungspartner (Proteine) 21.1, 21.2, ... übereinander ausgegeben. Für jeden der potentiellen Wechselwirkungspartner 21.1, 21.2, ... sind nur die Teilbereiche der zugehörigen Aminosäurenfolge, in denen eine mögliche Wechselwirkung mit der Abfragesequenz 20 erwartet werden kann. Hierbei sind die Teilbereiche (Kontaktbereiche) der möglichen Wechselwirkungspartner 21.1, 21.2, ..., die potentiell in Wechselwirkung mit der Abfragesequenz 20 bzw. deren Teilbereichen treten, mit Hilfe jeweiliger Balkenelemente unterhalb des Teilbereichs der Abfragesequenz 20 schematisch dargestellt, mit dem die jeweilige Wechselwirkung vorhergesagt worden ist. Es ist möglich, daß ein oder mehrere der potentiellen Wechselwirkungspartner 21.1, 21.2, ... jeweils mehrere Teilbereiche der zugehörigen Aminosäurenfolge aufweisen, die mit Teilbereichen der Abfragesequenz 20 potentiell in Wechselwirkung treten.

Wie aus Figur 2 hervorgeht, ermöglicht die beschriebene Verarbeitung und Ausgabe der elektronischen Daten aus der Speichereinrichtung 5 eine übersichtliche Auswertung für den Benutzer. Der Benutzer kann leicht Bereiche 22, 23 der Abfragesequenz 20 erkennen, in denen offensichtlich mit größerer Wahrscheinlichkeit eine Wechselwirkung stattfindet, als in anderen Bereichen 24 bzw. 25. Eine solche Auswertung auf der Basis der automatisch erzeugten, elektronischen Signale ist insbesondere dann möglich, wenn, wie dies in Figur 2 dargestellt ist, für eine große Anzahl von potentiellen Wechselwirkungsbereichen elektronische Signale ausgegeben werden.

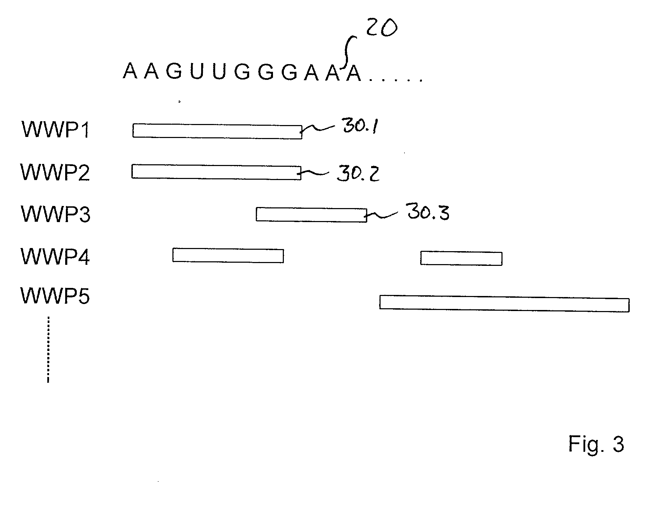

Figur 3 zeigt einen Teilbereich aus Figur 2 in einem größeren Maßstab. Unterhalb der Aminosäurenfolge der Abfragesequenz 20 sind einzelne Balkenelemente 30.1, 30.2, ... dargestellt, die Abschnitte der Aminosäurenfolge des zugehörigen Wechselwirkungspartners darstellen, dessen Kurzbezeichnung auf der linken Seite in Figur 3 ausgegeben ist.

Die in der vorstehenden Beschreibung, der Zeichnung und den Ansprüchen offenbarten Merkmale der Erfindung können sowohl einzeln als auch in beliebiger Kombination für die Verwirklichung der Erfindung in ihren verschiedenen Ausführungsformen von Bedeutung sein.

## Patentansprüche

1. Verfahren zum automatischen Verarbeiten und Ausgeben von elektronischen Daten aus einer Datenspeichereinrichtung mit Hilfe einer elektronischen Verarbeitungseinrichtung, wobei die elektronischen Daten eine in Teilfolgen unterteilbare Folge elektronischer Abfragedaten mit einer vorgegebenen Anordnung elektronischer Abfragedatenelemente und mehrere Folgen elektronischer Testdatenelemente mit einer jeweils vorgegebenen Anordnung elektronischer Testdaten umfassen, das Verfahren die folgenden Schritte aufweisend:
- Abrufen der Folge elektronischer Abfragedaten und der mehreren Folgen elektronischer Testdaten von der Datenspeichereinrichtung zu der elektronischen Verarbeitungseinrichtung;
- automatisches Verarbeiten der Folge elektronischer Abfragedaten und der mehreren Folgen elektronischer Testdaten in der elektronischen Verarbeitungseinrichtung, um automatisch zu erfassen, welcher Teilfolge der unterteilbaren Folge elektronischer Abfragedaten jede der mehreren Folgen elektronischer Testdaten jeweils zugeordnet ist;
- automatisches Erzeugen elektronischer Signale in Abhängigkeit von dem Ergebnis des Verfahrensschritts zum Feststellen, um in einer Ausgabeeinrichtung eine grafische Ausgabe automatisch zu erzeugen, bei der die mehreren Folgen elektronischer Testdaten jeweils in einem grafischen Ausgabeteilbereich dargestellt werden, welcher der jeweils zugeordneten Teilfolge der unterteilbaren Folge elektronischer Abfragedaten automatisch zugeteilt ist;
- Übermitteln der elektronischen Signale an die Ausgabeeinrichtung; und
- Erzeugen der grafischen Ausgabe auf der Basis der elektronischen Signale in der Ausgabeeinrichtung.

2. Verfahren nach Anspruch 1, **dadurch gekennzeichnet, daß** vor dem Verfahrensschritt zum automatischen Erzeugen der elektronischen Signale unter automatischer Berücksichtigung weiterer elektronischer Informationen eine wenigstens teilweise Überprüfung des Ergebnisses aus dem Verfahrensschritt zum Feststellen in der elektronischen Verarbeitungseinrichtung automatisch ausgeführt wird.

3. Verfahren nach Anspruch 2, **dadurch gekennzeichnet, daß** die weiteren elektronischen Informationen zumindest teilweise von externen Datenbanken abgerufen werden.

4. Verfahren nach einem der vorhergehenden Ansprüche, **dadurch gekennzeichnet, daß** die Folge elektronischer Abfragedaten eine elektronische Repräsentation einer Abfragegensequenz, die Teilfolgen elektronische Repräsentationen von Teilgensequenzen der Abfragegensequenz und die mehreren Folgen elektronischer Testdaten elektronische Repräsentationen von Abschnitten von Gensequenzen potentieller Wechselwirkungspartner der Abfragegensequenz sind.

5. Verfahren nach einem der vorhergehenden Ansprüche, **dadurch gekennzeichnet, daß** mit Hilfe der elektronischen Signale grafische Balkenelemente in dem grafischen Ausgabebereich erzeugt werden, die in mehreren Zeilen übereinander ausgegeben werden.

6. Vorrichtung zum automatischen Verarbeiten und Ausgeben von elektronischen Daten aus einer Datenspeichereinrichtung, wobei die elektronischen Daten eine in Teilfolgen unterteilbare Folge elektronischer Abfragedaten mit einer vorgegebenen Anordnung elektronischer Abfragedatenelemente und mehrere Folgen elektronischer Testdatenelemente mit einer jeweils vorgegebenen Anordnung elektronischer Testdaten umfassen, die Vorrichtung aufweisend:
- eine Abrufeinrichtung zum Abrufen der Folge elektronischer Abfragedaten und der mehreren Folgen elektronischer Testdaten von der Datenspeichereinrichtung;
- eine Prozessoreinrichtung zum automatischen Verarbeiten der Folge elektronischer Abfragedaten und der mehreren Folgen elektronischer Testdaten, um automatisch zu erfassen, welcher Teilfolge der unterteilbaren Folge elektronischer Abfragedaten jede der mehreren Folgen elektronischer Testdaten jeweils zugeordnet ist;
- eine von der Prozessoereinrichtung umfaßte Erzeugungseinrichtung zum automatischen Erzeugen elektronischer Signale in Abhängigkeit von dem Ergebnis des Feststellens, um in einer Ausgabeeinrichtung eine grafische Ausgabe automatisch zu erzeugen, bei der die mehreren Folgen elektronischer Testdaten jeweils in einem grafischen Ausgabeteilbereich dargestellt werden, welcher der jeweils zugeordneten Teilfolge der unterteilbaren Folge elektronischer Abfragedaten automatisch zugeteilt ist; und
- eine Übermittlungseinrichtung zum Übermitteln der elektronischen Signale an die Ausgabeeinrichtung.

7. Computerprogrammprodukt, welches in einen internen Speicher einer Computereinrichtung ladbar ist und welches Computerprogrammabschnitte zum Ausführen der Verfahrensschritte nach einem der Ansprüche 1 bis 5 beim Abarbeiten des Computerprogrammprodukts in der Computereinrichtung umfaßt.

8. Computerprogrammprodukt nach Anspruch 7, wobei wenigstens die Computerprogrammabschnitte auf einem von der Computereinrichtung lesbaren Speichermedium gespeichert sind.

9. Elektronische Datenstruktur, die mit Hilfe eines Verfahrens nach einem der Ansprüche 1 bis 5 gebildet wird und auf einem physikalischen Speicher gespeichert ist.
